**Europäisches Patentamt**

**European Patent Office**

**Office européen des brevets**

(11) Numéro de publication: **0 069 946**
**B1**

## (12) FASCICULE DE BREVET EUROPEEN

(45) Date de publication du fascicule du brevet:
23.04.86

(21) Numéro de dépôt: **82105947.4**

(22) Date de dépôt: **03.07.82**

(51) Int. Cl.⁴: **C 11 C 3/00**, C 07 C 69/533,
C 10 M 105/32, A 61 K 7/00

(54) **Huile, sa préparation et son utilisation, notamment dans les produits cosmétiques.**

(30) Priorité: **10.07.81 FR 8113820**

(43) Date de publication de la demande:
**19.01.83 Bulletin 83/3**

(45) Mention de la délivrance du brevet:
**23.04.86 Bulletin 86/17**

(84) Etats contractants désignés:
**AT BE CH DE FR GB IT LI LU NL SE**

(56) Documents cités:
**FR - A - 536 741**
**FR - A - 1 150 897**

**CHEMICAL ABSTRACTS, vol.60, 1964, colonne 8243b, Columbus, Ohio (US), J. BURESOVA et al.: "Preparation of esters of erucic acid with higher alcohols"**

(73) Titulaire: **CIRTA Centre International de Recherches et de Technologies Appliquées, Domaine de Clairval Boîte postale 75, F-74160 Collongés-sous-Salève (FR)**

(72) Inventeur: **Regnault, Alain, Villard Tacon Ornex, F-01210 Ferney-Voltaire (FR)**
Inventeur: **Michel, Jean-Pierre, 51, Avenue du Jura, F-01210 Ferney-Voltaire (FR)**
Inventeur: **Tournier, Hervé, Le Riondet, F-74520 Valleiry (FR)**

(74) Mandataire: **Moinas, Michel, Cabinet Michel Moinas 13 Chemin du Levant, F-01210 Ferney-Voltaire (FR)**

## Description

La présente invention se rapporte à une huile remplaçant avantageusement le blanc de baleine, le spermaceti de cachalot, le perhydrosqualène et l'huile de jojoba, ainsi qu'à un procédé de préparation de cette huile. L'invention a trait aussi à l'utilisation de cette huile, dans l'industrie de coupe des métaux, dans l'industrie pharmaceutique et particulièrement dans l'industrie cosmétique.

La raréfaction du blanc de baleine, du spermaceti de cachalot et du perhydrosqualène confère un intérêt grandissant aux esters d'acides gras et d'alcools gras monoinsaturés et d'une longueur de chaîne suffisante. Comme on le sait, les esters présentent des propriétés exceptionnelles par rapport aux triglycérides, qui font qu'ils sont utilisés comme matières premières dans les industries cosmétique et pharmaceutique et comme huile de coupe pour le travail des métaux.

Par ailleurs, on connaît d'après Chemical Abstracts 8243b (1964), un procédé de préparation d'érucylate d'oléyle à partir d'acide érucique et d'alcool oléique. Dans ce même article, on décrit des produits dont les propriétés se comparent à celles de la cire d'abeilles, par exemple un mélange d'acide érucique et d'érucylate de cétyle.

La principale source végétale connue d'esters gras est le jojoba, mais avec tous les aléas que présente un approvisionnement important en un produit extrait d'un arbuste vivant en zone semi-désertique dans le Sud-Ouest des Etats-Unis d'Amérique et le Nord-Ouest du Mexique.

En outre, la reconstitution de l'huile de jojoba est difficilement envisageable à un prix de revient satisfaisant pour des raisons de disponibilité de matières premières, cette huile contenant des acides gras et des alcools gras rares.

L'invention a précisément pour objectif de pallier les difficultés d'approvisionnement en huile de jojoba et l'impossibilité pratique de reconstituer celle-ci.

L'invention en question a trait à une huile constituée essentiellement d'érucylate d'oléyle et/ou d'oléate d'érucyle et contenant également de l'érucylate d'érucyle et/ou de l'oléate d'oléyle, en % pondéraux comme suit:

| | |
|---|---|
| érucylate d'oléyle et/ou oléate d'érucyle ($C_{40}$) | 33 à 76% |
| ( érucylate d'érucyle ($C_{44}$) | 0 à 18% |
| oléate d'oléyle ($C_{36}$) | 15 à 35% |

Ces huiles ont un point de trouble compris entre 15 et 18 °C. Elles sont liquides à température ambiante (22 °C).

Bien qu'ayant des vertus, en cosmétique notamment, comparables à celles de l'huile de jojoba, l'huile selon l'invention s'en distingue radicalement.

En effet, elle contient des esters en $C_{40}$ di-insaturés, présentant une insaturation sur la partie alcool et une insaturation sur la partie acide («di-mono-insaturé»):

érucylate d'oléyle = docosénoate d'octadécényle $C_{22}$ + $C_{18}$

oléate d'érucyle = octadécénoate de docosényle $C_{18}$ + $C_{22}$

qui n'ont pas été identifiés dans l'huile de jojoba, tandis que les esters «di-mono-insaturés» en $C_{36}$ et $C_{44}$:

oléate d'oléyle = octadécénoate d'octadécényle $C_{18}$ + $C_{18}$

érucylate d'érucyle = docosénoate de docosényle $C_{22}$ + $C_{22}$

n'en sont que des constituants très mineurs

Inversement, les constituants majeurs de l'huile de jojoba, qui sont en $C_{42}$ et $C_{40}$:

| | |
|---|---|
| éicosénoate de docosényle | $C_{20}$ + $C_{22}$ |
| éicosénoate d'éicosényle | $C_{20}$ + $C_{20}$ |

n'entrent pas dans la constitution de l'huile selon l'invention, ou seulement en proportions non significatives.

Les acides oléiques et éruciques sont, à l'état de glycérides, les principaux constituants de l'huile de colza, huile alimentaire très répandue. On a l'habitude d'éliminer l'acide érucique de l'huile de colza destinée à un usage alimentaire et de ce fait, il se retrouve en grande quantité sur le marché comme sous-produit dont on ne sait trop que faire.

L'huile selon l'invention est donc un moyen de valoriser ce sous-produit et, indirectement, l'huile de colza.

Plus généralement, ces acides se rencontrent en quantités appréciables dans les plantes tropaélacées et crucifères, comme glycérides, habituellement dans les graines. Ces sources naturelles peuvent être utilisées au même titre que le colza.

Citons parmi les tropaelacées la capucine (Tropaelonum); parmi les crucifères, outre le colza (Brassica, Eruca), le radis (Raphanus), le cresson (Nasturtium) le raifort (Armoracia), les diverses moutardes (Brassica, Sisymbrium, Coringa, Sinapis, Alliuria), Crambe, Cruscastrum, Thlaspi, etc.

La préparation de l'huile selon l'invention peut, par exemple, être effectuée en présence d'un catalyseur acide et sans solvant, en faisant réagir l'acide érucique et/ou oléique avec l'alcool oléique et/ou érucique.

L'invention se rapporte également à un procédé de préparation de l'huile à partir de plantes riches en acide érucique, les tropaelacées et les crucifères. Selon ce procédé, on hydrolyse par saponification les glycérides contenus dans ces plantes, on sépare l'hydrolysat en deux parties sensiblement égales, on traite une des parties par réduction de façon à transformer les acides gras en alcool gras, on fait réagir la partie réduite sur l'autre partie non réduite pour estérifier les alcools gras par les acides et enfin on isole l'huile ainsi obtenue.

En variante, on peut partir de deux lots de glycérides sensiblement égaux et procéder à la réduction directement sur un des lots de glycérides.

Après saponification des glycérides, on peut travailler directement sur les mélanges acides érucique et oléique tels qu'obtenus, ou bien on peut isoler l'acide érucique et l'acide oléique l'un de l'autre ou encore en modifier les proportions respectives par cristallisation fractionnée. Celle-ci permet d'ailleurs un premier stade de purification.

Les alcools insaturés correspondants, à savoir l'alcool oléique et l'alcool érucique, seuls ou en mélange, peuvent être préparés par action du sodium dans l'alcool ou de l'hydrure de lithium et d'aluminium sur les acides ou, selon la variante, directement sur les glycérides. Pour le traitement de grandes quantités, on préfère procéder à la réduction catalytique des glycérides, sous pression, en présence de chromite de zinc comme catalyseur.

La préparation de l'huile, selon l'invention, par estérification des couples alcools/acides ci-dessus, se fait avantageusement sans solvant et en présence d'un catalyseur acide, tel que l'acide para-toluène-sulfonique. On opère de préférence à une température comprise entre 120 et 160 °C sous pression réduite, de l'ordre de 60 à 600 mbars pour distiller l'eau d'estérification. Dans ces conditions, le temps de réaction est typiquement compris entre une et quatre heures.

Selon les alcools et acides gras de départ mis en œuvre, les produits obtenus sont essentiellement de l'érucylate d'oléyle, de l'oléate d'érucyle (tous deux $C_{40}$) ou leur mélange, accompagnés d'oléate d'oléyle ($C_{36}$) et d'érucylate d'érucyle ($C_{44}$) séparément ou en mélange. Les éventuelles impuretés provenant des produits de départ, tels que le stéarate et le palmitate d'érucyle ou d'oléyle, sont avantageusement éliminées, par exemple, par cristallisation fractionnée.

A chaque étape, on peut d'ailleurs procéder à une purification, par cristallisation fractionnée, passage sur C actif et, si nécessaire, désodorisation par entraînement à la vapeur d'eau.

L'huile peut généralement être utilisée telle quelle, car elle ne présente pas d'odeur ni de couleur prononcée; elle est blanche ou légèrement jaune. Si désiré cependant, on peut procéder à un ultime raffinage sur C actif et/ou par entraînement à la vapeur d'eau.

Elle se substitue particulièrement bien aux esters d'acides gras et d'alcools gras mono-insaturés obtenus par extraction d'autres sources naturelles. Elle remplace donc notamment le blanc de baleine, le spermaceti de cachalot, le perhydrosqualène et l'huile de jojoba dans leurs applications cosmétique, pharmaceutique et dans le travail des métaux.

Par exemple, on peut utiliser l'huile dans des produits cosmétiques de nature fort diverse, tels que des crèmes, des émulsions, des huiles, des lotions, des laits, etc.

Les exemples suivants illustrent l'invention, les exemples 4 à 16 ayant plus particulièrement trait aux utilisations en cosmétique.

Exemple 1

On place dans un réacteur en acier inoxydable de 1000 litres muni d'un système d'agitation mécanique et d'un condenseur environ:
– 350 kg d'acide érucique commercial (indice d'acide: 165 à 175, indice d'iode: 70 à 76) titrant 85 à 90% d'acide érucique, le reste étant pour l'essentiel de l'acide oléique.
– 286 kg d'alcool oléïque commercial (indice d'hydroxyle: 205 à 215, indice d'iode: 90–96) titrant 85 à 90% d'alcool oléïque;
– 2 kg d'acide para-toluène sulfonique.

Les quantités exactes d'acide et d'alcool mises en réaction sont déterminées à chaque fois sur la base des indices d'acide et d'hydroxyle des matières premières de façon à conserver un rapport équimoléculaire entre l'acide et l'alcool. Le catalyseur est introduit à raison de 1% molaire par rapport à l'acide gras.

On purge le réacteur à l'azote et on porte le mélange réactionnel à 140 °C sous pression réduite (150 à 250 mbars) en maintenant une agitation mécanique jusqu'à ce que l'eau de condensation soit recueillie au fond du condenseur (environ 19 litres) et que l'indice d'acide correspondant à l'acide gras soit inférieur à 3.

On ramène alors la température à 50 °C et on procède à 3 ou 4 lavages sous agitation vigoureuse avec 200 litres d'eau par lavage pour éliminer l'acide paratoluène-sulfonique, c'est-à-dire jusqu'à ce que l'indice d'acide total soit inférieur à 3.

On décante soigneusement l'eau et on décolore l'huile sur charbon actif et terres décolorantes.

Si la teneur totale en acide et en alcool saturés dépasse 10 à 15%, le point de trouble est supérieur de 17 à 18 °C. On laisse alors reposer l'huile pendant 48 heures en la maintenant entre 15 et 18 °C et on élimine ensuite par filtration le précipité essentiellement constitué d'ester gras mono-insaturés comme le montre l'indice d'iode (indice d'iode de l'ordre de 40 à 50):

Le rendement moyen en huile purifiée est de 87% molaire, soit environ 540 kg. Cette huile a les caractéristiques suivantes:

| | |
|---|---|
| Indice d'acide: | < 1 |
| Indice de péroxyde: | < 2 |
| Indice de saponification: | 95–105 |
| Indice d'iode: | 82–88 |

Exemple 2

On place dans un réacteur en acide inoxydable de 1000 litres muni d'un système d'agitation mécanique et d'un condenseur environ:
– 350 kg d'une coupe oléo-érucique provenant du raffinage de l'huile de colza, titrant environ 40% d'acide oléïque, 10% d'acide eicosénoïque; 40% d'acide érucique et 10% d'acides palmitique et stéarique (indice d'acide: 180 à 190, indice d'iode: 72 à 76);
– 309 kg d'alcool oléïque commercial (indice d'hydroxyle: 205 à 215, indice d'iode: 90 à 96) titrant 85 à 90% d'alcool oléïque;
– 2 kg d'acide para-toluène sulfonique.

Les quantités exactes d'acide et d'alcool mises en réaction sont déterminées à chaque fois sur la base des indices d'acide et d'hydroxyle des matières premières de façon à conserver un rapport équimoléculaire entre l'acide et l'alcool. Le catalyseur est introduit à raison de 1% molaire par rapport à l'acide gras.

On procède ensuite comme décrit à l'exemple 3.

Le rendement moyen en huile purifié est de 85% molaire, soit environ 550 kg. Cette huile a les caractéristiques suivantes:

| | |
|---|---|
| Indice d'acide: | < 1 |
| Indice de péroxyde: | < 2 |
| Indice de saponification: | 100–110 |
| Indice d'iode: | 86–92 |

Exemple 3

On place dans un réacteur en acier inoxydable de 1000 litres muni d'un système d'agitation mécanique et d'un condenseur environ:

– 350 kg d'acide oléïque commercial (indice d'acide: 190 à 200, indice d'iode: 88 à 94) titrant 80 à 85% d'acide oléïque;

– 395 kg d'alcool érucique commercial (indice d'hydroxyle: 160 à 170, indice d'iode: 78 à 84) titrant 80 à 85% d'alcool érucique obtenu par réduction de l'acide érucique commercial, le reste étant pour l'essentiel de l'alcool oléïque.

– 2,4 kg d'acide para-toluène sulfonique.

Les quantités exactes d'acide et d'alcool mises en réaction sont déterminées à chaque fois sur la base des indices d'acide et d'hydroxyle des matières premières de façon à conserver un rapport équimoléculaire entre l'acide et l'alcool. Le catalyseur est introduit à raison de 1% molaire par rapport à l'acide gras.

On procède ensuite comme décrit à l'exemple 1. Le rendement moyen en huile purifiée est de 85% molaire, soit environ 620 kg. Cette huile a les caractéristiques suivantes:

| | |
|---|---|
| Indice d'acide: | < 1 |
| Indice de péroxyde: | < 2 |
| Indice de saponification: | 95–105 |
| Indice d'iode: | 84–90 |

Exemple 4

Crème nourrissante au collagène:

| Position | % | Produit |
|---|---|---|
| 1 | 6,0 | cire d'abeilles |
| 2 | 8,0 | vaseline |
| 3 | 6,0 | huile selon l'invention |
| 4 | 3,0 | extrait de camomille soluble dans l'huile |
| 5 | 0,1 | antioxydant |
| 6 | 1,0 | huile de tournesol |
| 7 | 7,5 | émulgateur |
| 8 | 1,0 | ester de glycérine de la vitamine F |
| 9 | 0,4 | ®Lipoconserv |
| 10 | 2,5 | sorbitol 70% |
| 11 | 0,3 | sulfate de magnésium |
| 12 | 6,3 | borax |
| 13 | 2,0 | agent d'humidité |
| 14 | 0,2 | ®Hydroconserv, agent anti-microbien |
| 15 | 2,0 | extrait de ginseng |
| 16 | 1,0 | extrait d'arnica |
| 17 | 52,2 | eau chaude |
| 18 | 0,5 | parfum |
| 19 | 5,0 | collagène natif |
| 20 | 1,0 | extrait de plante aquoglycolique |

Mode opératoire:
A) Chauffer ensemble les positions 1 à 9 et mélanger.
B) Dissoudre les positions 10 à 16 dans la position 17 et chauffer à 70°C.
C) Ajouter la solution B) au mélange fondu A) en les agitant, homogénéiser et agiter à froid.
D) Ajouter les positions 18 à 20.

Exemple 5

Emulsion hydratante

| Position | % | Produit |
|---|---|---|
| 1 | 5,0 | émulgateur |
| 2 | 4,0 | huile d'arachide |
| 3 | 2,0 | huile d'amande |
| 4 | 3,0 | huile selon l'invention |
| 5 | 2,0 | ester de glycérine de la vitamine F |

| Position | % | Produit |
|---|---|---|
| 6 | 0,1 | huile de silicium |
| 7 | 0,2 | ®lipoconserv |
| 8 | 0,05 | ®Oxynex Cos |
| 9 | 0,2 | ®Hydroconserv |
| 10 | 1,5 | sorbitol 70% |
| 11 | 69,65 | eau très chaude |
| 12 | 3,0 | extrait d'aloès |
| 13 | 3,0 | collagène natif pur |
| 14 | 6,0 | agent d'humidité |
| 15 | 0,3 | parfum |

A) Chauffer à 70°C les positions 1 à 8 et faire fondre.

B) Dissoudre les positions 9 et 10 dans 11 et chauffer à 70°C.

C) Ajouter et agiter la solution B) au mélange fondu et émulsionner.

D) Agiter et refroidir les positions 12 à 15 à 35°C, les ajouter et continuer à refroidir jusqu'à la température ambiante.

## Exemple 6

### Crème douce au collagène

| Position | % | Produit |
|---|---|---|
| 1 | 13,3 | ®Lamecrème LPM |
| 2 | 0,7 | ®Lamacit GML 20 |
| 3 | 3,3 | lanoline |
| 4 | 4,7 | spermaceti |
| 5 | 4,0 | huile d'avocat |
| 6 | 0,7 | huile de silicone |
| 7 | 9,0 | huile de vaseline |
| 8 | 0,4 | ®Lipoconserv |
| 9 | 3,0 | ®Miglyol |
| 10 | 4,3 | huile selon l'invention |
| 11 | 0,3 | ®Hydroconserv |
| 12 | 5,0 | Glycérine |
| 13 | 45,2 | eau |
| 14 | 6,0 | collagène natif |
| 15 | 0,1 | parfum |

A) Fondre les positions 1 à 10 et chauffer à 70°C.

B) Dissoudre les positions 11 et 12 dans la position 13 et chauffer à 70°C.

C) Ajouter la solution B) au mélange fondu A) et mélanger soigneusement. Refroidir en agitant jusqu'à 35°C et ajouter alors les positions 14 et 15. Continuer à agiter et refroidir jusqu'à la température ambiante.

## Exemple 7

### Huile pour les cheveux Sauna/Fitness

| Position | % | Produit |
|---|---|---|
| 1 | 15,0 | huile d'amande |
| 2 | 25,0 | myristate d'isopropyle |
| 3 | 7,5 | huile selon l'invention |
| 4 | 25,0 | huile d'arachide |
| 5 | 5,0 | extrait de bouleau soluble dans l'huile |
| 6 | 22,0 | huile d'olive |
| 7 | 0,5 | huile de parfum |
| 8 | 0,05 | ®Oxynex 2004 |

A) Dissoudre la position 7 dans une partie de la position 2 en chauffant.
B) Ajouter cette solution sous agitation au mélange formé par les positions 1 à 5.
C) Enfin, ajouter la position 6 et bien mélanger tout en ajoutant la position 8.

### Exemple 8

#### Emulsion après soleil pour peau sèche

| Position | % | Produit |
|---|---|---|
| 1 | 5,0 | émulgateur |
| 2 | 1,0 | huile de carotte |
| 3 | 3,0 | ester de glycérine de la vitamine F |
| 4 | 0,05 | antioxydant |
| 5 | 2,0 | huile selon l'invention |
| 6 | 2,5 | alcool oléïque |
| 7 | 3,0 | graisse d'arachide |
| 8 | 2,0 | myristate d'isopropyle |
| 9 | 1,0 | ®Solulan 98 |
| 10 | 1,5 | sorbitol 70% |
| 11 | 1,0 | glycérine |
| 12 | 0,02 | acide citrique |
| 13 | 0,1 | acide sorbique |
| 14 | 0,25 | ®Hydroconserv |
| 15 | 64,8 | eau |
| 16 | 3,5 | collagène natif |
| 17 | 6,0 | agent d'humidité |
| 18 | 0,23 | panthénol |
| 19 | 0,05 | parfum |
| 20 | 1,5 | extrait de germe de blé |
| 21 | 1,5 | extrait de plante aquoglycolique |

A) Fondre les positions 1 à 8 à 70 °C.
B) Dissoudre les positions 9 à 14 dans la position 15 et chauffer la solution à 70 °C.
C) Ajouter la solution B au mélange fondu A) et émulsionner.

D) Agiter et refroidir jusqu'à 35 °C et ajouter alors les positions 16 à 21, puis continuer à refroidir jusqu'à la température ambiante.

### Exemple 9

#### Lotion corporelle Sauna/Fitness

| Position | % | Produit |
|---|---|---|
| 1 | 4,0 | émulgateur |
| 2 | 5,0 | myristate d'isopropyle |
| 3 | 3,0 | graisse d'arachide |
| 4 | 3,0 | huile selon l'invention |
| 5 | 0,2 | ®Lipoconserv, agent de conservation |
| 6 | 0,1 | huile de silicone |
| 7 | 2,5 | sorbitol 70% |
| 8 | 0,2 | allantoïne |
| 9 | 0,1 | ®Hydroconserv |
| 10 | 71,7 | eau et solution colorée |
| 11 | 5,0 | agent d'humidité |
| 12 | 1,0 | extrait de bouleau |
| 13 | 3,0 | collagène natif |
| 14 | 0,7 | extrait aquo-glycolique de plante |
| 15 | 0,5 | parfum |

A) Fondre les positions 1 à 6 et chauffer à 70 °C.
B) Dissoudre les positions 7 à 9 dans la position 10 et chauffer à 70 °C.
C) Ajouter la solution B) au mélange fondu A) et mélanger soigneusement. Agiter et refroidir jusqu'à 35 °C et ajouter alors les positions 11 à 15. Continuer à agiter et refroidir jusqu'à la température ambiante.

Exemple 10

### Lait de toilette

| Position | % | Produit |
|---|---|---|
| 1 | 3,0 | huile d'arachide |
| 2 | 5,0 | huile de vaseline |
| 3 | 5,0 | émulgateur |
| 4 | 3,0 | huile selon l'invention |
| 5 | 2,0 | ester de glycérine de vitamine F |
| 6 | 0,2 | ®Lipoconserv, agent de conservation |
| 7 | 0,05 | ®Oxynex 2004 |
| 8 | 0,15 | ®Hydroconserv |
| 9 | 75,4 | eau |
| 10 | 0,2 | parfum |
| 11 | 3,0 | agent d'humidité |
| 12 | 2,0 | extrait d'aloès |
| 13 | 1,0 | extrait de millepertuis |

A) Chauffer les positions 1 à 7 à 70°C et faire fondre.

B) Dissoudre la position 8 dans la position 9 et chauffer à 70°C.

C) Ajouter la solution B au mélange fondu A) en agitant et émulsionner.

D) Agiter et refroidir, ajouter les positions 10 à 13 à 35°C et continuer à agiter jusqu'à la température ambiante.

Exemple 11

### Crème de massage

| Position | % | Produit |
|---|---|---|
| 1 | 2,0 | émulgateur |
| 2 | 0,5 | ®Cremophor |
| 3 | 3,0 | spemaceti |
| 4 | 8,0 | alcool cétyl-stéarique |
| 5 | 10,0 | huile de vaseline |
| 6 | 5,0 | huile selon l'invention |
| 7 | 5,0 | ester de glycérine de la vitamine F |
| 8 | 0,3 | ®Lipoconserv, agent de conservation |
| 9 | 53,9 | eau |
| 10 | 8,0 | propylenglycol |
| 11 | 5,0 | agent d'humidité |
| 12 | 0,2 | parfum |

A) Chauffer les positions 1 à 8 à 70°C et faire fondre.

B) Dissoudre la position 9 dans la position 8 et chauffer à 70°C.

C) Ajouter la solution B) au mélange fondu A) en agitant et émulsionner.

D) Agiter à froid et ajouter les positions 10 à 12 à 35°C environ.

Exemple 12

### Crème pour les mains avec agent hydratant

| Position | % | Produit |
|---|---|---|
| 1 | 10,0 | acide stéarique |
| 2 | 1,0 | alcool cétylique |
| 3 | 5,0 | monostéarate de glycérine |
| 4 | 5,0 | myristate d'isopropyle |
| 5 | 6,5 | huile selon l'invention |
| 6 | 0,3 | ®Lipoconserv, agent de conservation |

| Position | % | Produit |
|---|---|---|
| 7 | 3,0 | amphisol |
| 8 | 3,0 | sorbitol 70% |
| 9 | 3,0 | propylenglycol |
| 10 | 54,7 | eau |
| 11 | 5,0 | agent d'humidité |
| 12 | 0,5 | parfum |
| 13 | 2,0 | extrait de camomille |
| 14 | 1,0 | extrait d'arnica |

A) Faire fondre les positions 1 à 5 et chauffer à 70 °C.

B) Dissoudre les positions 6 et 7 dans le mélange fondu.

C) Dissoudre les positions 8 et 9 dans la position 10 et chauffer à 70 °C.

D) Ajouter la solution C) au mélange fondu en agitant et émulsionner.

E) Agiter et refroidir. Ajouter les positions 11 à 14 à 35 °C et continuer à refroidir jusqu'à la température ambiante.

### Exemple 13

**Emulsion après-rasage**

| Position | % | Produit |
|---|---|---|
| 1 | 4,0 | émulgateur |
| 2 | 6,0 | myristate d'isopropyle |
| 3 | 1,0 | graisse d'arachide |
| 4 | 2,0 | huile selon l'invention |
| 5 | 0,2 | ®Lipoconserv, agent de conservation |
| 6 | 0,1 | huile de silicone |
| 7 | 2,0 | sorbitol 70% |
| 8 | 0,2 | allantoine |
| 9 | 0,1 | ®Hydroconserv |
| 10 | 70,0 | eau |
| 11 | 8,0 | distillat d'hamamélis |
| 12 | 1,0 | extrait d'aquo-glycolique de plante |
| 13 | 3,0 | agent d'humidité |
| 14 | 2,0 | collagène natif |
| 15 | 0,5 | parfum |

A) Chauffer les positions 1 à 6 à 70 °C et faire fondre.

B) Dissoudre les positions 7 à 9 dans la position 10 et chauffer à 70 °C.

C) Ajouter la solution B) au mélange fondu A) et émulsionner.

D) Agiter et refroidir, ajouter les positions 11 à 15 à 35 °C et continuer à refroidir.

### Exemple 14

**Masque pour le visage**

| Position | % | Produit |
|---|---|---|
| 1 | 2,5 | émulgateur |
| 2 | 0,5 | huile de carotte |
| 3 | 1,0 | huile selon l'invention |
| 4 | 1,5 | graisse d'arachide |
| 5 | 2,0 | pristane |
| 6 | 1,0 | alcool oléïque |
| 7 | 0,1 | antioxidant |
| 8 | 0,2 | ®Lipoconserv, agent de conservation |
| 9 | 2,0 | myristate d'isopropyle |
| 10 | 0,4 | tylose H–4000 |
| 11 | 0,2 | ®Hydroconserv |
| 12 | 2,0 | sorbitol 70% |
| 13 | 40,4 | eau |

| Position | % | Produit |
|---|---|---|
| 14 | 5,0 | collagène natif |
| 15 | 2,0 | agent d'humidité |
| 16 | 2,0 | hydrolysat d'élastine |
| 17 | 2,0 | extrait aquo-glycolique de plante |
| 18 | 3,0 | extrait de rose |
| 19 | 2,0 | extrait d'aristoloche |
| 20 | 0,2 | huile de parfum |
| 21 | 30,0 | kaolin |

A) Chauffer les positions 1 à 9 à 70 °C et faire fondre.

B) Dissoudre les positions 10 à 12 dans la position 13 et chauffer à 70 °C.

C) Ajouter la solution B) au mélange A) en agitant, émulsionner et agiter à froid, ajouter les positions 14 à 20 à 35 °C et bien agiter.

D) Malaxer soigneusement l'émulsion obtenue avec la position 21 jusqu'à ce qu'on obtienne une masse uniforme.

### Exemple 15

| | Crème de jour au collagène pour peau sensible | |
|---|---|---|
| Position | % | Produit |
| 1 | 8,0 | acide stéarique |
| 2 | 2,0 | monostéarat de glycérine |
| 3 | 5,0 | huile de carotène |
| 4 | 2,0 | ester de glycérine de la vitamine F |
| 5 | 2,0 | huile de carotte |
| 6 | 2,0 | émulgateur |
| 7 | 0,1 | huile de silicone |
| 8 | 3,0 | huile selon l'invention |
| 9 | 3,0 | beurre d'arachide |
| 10 | 0,05 | ®Oxynex 2004 |
| 11 | 0,3 | ®Lipoconserv, agent de conservation |
| 12 | 2,0 | sorbitol 70% |
| 13 | 1,0 | amino-methyl-propanol |
| 14 | 0,15 | ®Hydroconserv |
| 15 | 0,2 | allantoine |
| 16 | 57,9 | eau |
| 17 | 3,0 | agent d'humidité |
| 18 | 5,0 | collagène natif |
| 19 | 2,0 | distillat d'hamamélis |
| 20 | 1,0 | extrait aquo-glycolique de plante |
| 21 | 0,3 | parfum |

A) Chauffer les positions 1 à 11 à 70 °C et faire fondre.

B) Dissoudre les positions 12 à 15 dans la position 16 et chauffer à 70 °C.

C) Ajouter la solution B) au mélange fondu A) en agitant et émulsionner.

D) Agiter et refroidir, ajouter les positions 17 à 21 à 35 °C et continuer à refroidir jusqu'à la température ambiante.

### Exemple 16

| | Crème nourrissante au collagène | |
|---|---|---|
| Position | % | Produit |
| 1 | 6,0 | cire d'abeille |
| 2 | 8,0 | vaseline |
| 3 | 6,0 | huile selon l'invention |
| 4 | 3,0 | extrait de camomille, soluble dans l'huile |
| 5 | 0,1 | antioxydant |
| 6 | 1,0 | huile de tournesol |

| Position | % | Produit |
|---|---|---|
| 7 | 7,5 | émulgateur |
| 8 | 1,0 | ester de glycérine de la vitamine F |
| 9 | 0,4 | ®Lipoconserv, agent de conservation |
| 10 | 2,5 | sorbitol 70% |
| 11 | 0,3 | sulfate de magnésium |
| 12 | 0,3 | borax |
| 13 | 2,0 | agent d'humidité |
| 14 | 0,2 | ®Hydroconserv |
| 15 | 2,0 | extrait de ginseng |
| 16 | 1,0 | extrait d'arnica |
| 17 | 52,2 | eau très chaude |
| 18 | 0,5 | parfum |
| 19 | 5,0 | collagène natif |
| 20 | 1,0 | extrait aquo-glycolique de plante |

A) Chauffer les positions 1 à 9 à 70°C et faire fondre.

B) Dissoudre les positions 10 à 16 dans la position 17 et chauffer à 70°C.

C) Ajouter la solution B) au mélange fondu A) en agitant, homogénéiser et agiter à froid.

D) Ajouter les positions 18 à 20 à environ 35°C.

## Revendications

1. Huile constituée essentiellement d'érucylate d'oléyle et/ou d'oléate d'érucyle, caractérisée en ce qu'elle contient de l'érucylate d'érucyle et/ou de l'oléate d'oléyle, en % pondéraux comme suit:

| | |
|---|---|
| érucyclate d'oléyle et/ou oléate d'érucyle | 33 à 76% |
| érucyclate d'érucyle | 0 à 18% |
| oléate d'oléyle | 15 à 35% |

2. Huile selon la revendication 1, caractérisée en ce que son point de trouble est compris entre 15 et 18°C.

3. Procédé de préparation de l'huile selon la revendication 1, caractérisée en ce qu'on isole, à partir des glycérides de plantes tropaelacées ou crucifères, l'acide érucique et/ou oléïque d'une part, l'alcool oléïque et/ou érucique d'autre part, et qu'on fait réagir les acides avec les alcools, en présence d'un catalyseur et sans solvant.

4. Procédé de préparation de l'huile selon la revendication 3, caractérisé en ce que la plante est le colza.

5. Utilisation de l'huile selon la revendication 1 comme huile de coupe pour le travail des métaux.

6. Utilisation de l'huile selon la revendication 1 dans l'industrie pharmaceutique.

7. Utilisation de l'huile selon la revendication 1 dans l'industrie cosmétique.

8. Utilisation selon la revendication 7, à raison de 1 à 7,5% en poids dans les produits cosmétiques.

## Claims

1. An oil consisting essentially of oleyl erucylate and/or erucyl oleate, characterized in that it contains erucyl erucylate and/or oleyl oleate, in % by weight:

| | |
|---|---|
| oleyl erucylate and/or erucyl oleate | 33 to 76% |
| erucyl erucylate | 0 to 18% |
| oleyl oleate | 15 to 35% |

2. An oil according to Claim 1, characterized in that its cloud point is from 15 to 18°C.

3. A process of preparation of the oil according to Claim 1, characterized in that erucic and/or oleic acid on the one hand and oleic and/or erucic alcohol on the other hand are isolated from glycerides of Tropoelacae or Cruciferous plants, and that the acids are made to react with the alcohols in the presence of a catalyst and without a solvent.

4. A process of preparation of the oil according to Claim 3, characterized in that the plant is colza.

5. The use of the oil according to Claim 1, as a cutting oil for metals.

6. The use of the oil according to Claim 1, in the pharmaceutical industry.

7. The use of the oil according to Claim 1, in the cosmetics industry.

8. The use according to Claim 7, in the amount of 1 to 7.5% by weight in cosmetic products.

## Patentansprüche

1. Öl, im wesentlichen bestehend aus Erucasäure-oleylester und/oder Ölsäure-erucylester, dadurch gekennzeichnet, dass es Erucasäure-erucylester und/oder Ölsäure-oleylester mit folgenden Gewichtsprozenten enthält:

| | |
|---|---|
| Erucasäureoleylester und/oder Ölsäureerucylester | 33 bis 76% |
| Erucasäureerucylester | 0 bis 18% |
| Ölsäureoleylester | 15 bis 35% |

2. Öl nach Anspruch 1, dadurch gekennzeichnet, dass sein Trübungspunkt zwischen 15 und 18°C liegt.

3. Verfahren zur Herstellung des Öls nach Anspruch 1, dadurch gekennzeichnet, dass man aus den Glyceriden von Tropaelacea- oder Kreuzblütlerpflanzen einerseits die Eruca- und/oder Ölsäure und andererseits Oleyl- und/oder Erucylalkohol isoliert und dann die Säuren in Gegenwart eines Katalysators lösungsmittelfrei mit den Alkoholen umsetzt.

4. Verfahren nach Anspruch 3, dadurch gekennzeichnet, dass die Pflanze Raps ist.

5. Verwendung des Öls nach Anspruch 1 als Schneidöl bei der Metallbearbeitung.

6. Verwendung des Öls nach Anspruch 1 in der Heilmittelindustrie.

7. Verwendung des Öls nach Anspruch 1 in der Kosmetikindustrie.

8. Verwendung nach Anspruch 7 in Anteilen von 1 bis 7,5 Gew.-% in Körperpflegeerzeugnissen.